(19) Europäisches Patentamt
European Patent Office
Office européen des brevets

(11) Veröffentlichungsnummer: **0 033 980**
**A1**

# (12) EUROPÄISCHE PATENTANMELDUNG

(21) Anmeldenummer: **81100990.1**

(22) Anmeldetag: **12.02.81**

(51) Int. Cl.³: **C 07 C 59/68**, C 07 C 69/712, A 61 K 31/19, A 61 K 31/215, C 07 C 51/00, C 07 C 67/00

(30) Priorität: **12.02.80 IT 333480**

(43) Veröffentlichungstag der Anmeldung: **19.08.81**
**Patentblatt 81/33**

(84) Benannte Vertragsstaaten: **BE CH DE FR GB LI NL SE**

(71) Anmelder: **SCHIAPPARELLI FARMACEUTICI S.p.A., Corso Belgio 86, I-10153 Torino (IT)**

(72) Erfinder: **Golinelli, Marino, Piazza di Porta San Mamolo, 1-20, I-40136 Bologna (IT)**

(74) Vertreter: **Beszédes, Stephan G. Dr., Münchener Strasse 80a Postfach 1168, D-8060 Dachau (DE)**

(54) **2-Methylpropionsäurederivate, Verfahren zur Herstellung derselben und diese enthaltende Arzneimittel.**

(57) Die Anmeldung betrifft 2-Methylpropionsäurederivate der allgemeinen Formel

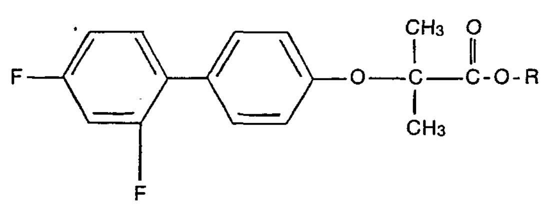

I

worin
R für Wasserstoff oder einen Alkylrest mit 1 bis 8 Kohlenstoffatom(en) steht
sowie ein Verfahren zu ihrer Herstellung.

Diese Verbindungen können in Arzneimitteln auf dem Gebiet der Therapie, insbesondere gegen Hyperlipidämie und Hypercholesterinämie, verwendet werden.

DR. STEPHAN G. BESZÉDES
PATENTANWALT

ZUGELASSENER VERTRETER
AUCH BEIM EUROPÄISCHEN PATENTAMT

PROFESSIONAL REPRESENTATIVE ALSO
BEFORE THE EUROPEAN PATENT OFFICE

8060 DACHAU BEI MÜNCHEN
POSTFACH 1168

MÜNCHENER STRASSE 80 A

Bundesrepublik Deutschland

TELEPHON DACHAU 4371
Postscheckkonto München (BLZ 700 100 80)
Konto-Nr. 1 368 71
Bankkonto-Nr. 906 370 bei der Kreis- und Stadtsparkasse Dachau-Indersdorf (BLZ 700 515 40)
(VIA Bayerische Landesbank
Girozentrale, München)

1 419 EU

B e s c h r e i b u n g

zur Patentanmeldung

SCHIAPPARELLI FARMACEUTICI S.p.A.

Torino, Italien

betreffend

2-Methylpropionsäurederivate, Verfahren zur Herstellung derselben und diese enthaltende Arzneimittel

Die Erfindung betrifft neue 2-Methylpropionsäurederivate, ein Verfahren zur Herstellung derselben sowie diese Verbindungen enthaltende Arzneimittel, insbesondere

solche mit den Lipidgehalt und den Cholesteringehalt im Blut senkender (hypolipidämischer und hypocholesterinämischer) Wirkung.

Es sind bereits zur Verminderung des Lipid- und Cholesteringehaltes im Blut fähige Verbindungen aus dem Schrifttum bekannt. Beispielsweise sind in der US-Patentschrift 3 262 850 Verbindungen mit einem Teil der Formel

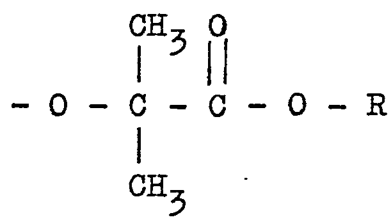

,

dessen Sauerstoffatom stets an einen, gegebenenfalls durch Halogen, Alkyl- oder Alkoxyreste substituierten, Phenylrest gebunden ist, beschrieben. Aus der belgischen Patentschrift 790 026 sind ähnliche Verbindungen, deren Substituenten am an das Sauerstoffatom der obigen Gruppe gebundenen Phenylring jedoch im allgemeinen Acylreste, wie p-Chlorbenzoylreste, sind, bekannt.

Der Erfindung liegt die Aufgabe zugrunde, neue 2-Methylpropionsäurederivate mit überlegenen pharmakologischen, insbesondere den Lipidgehalt und Cholesteringehalt im Blut senkenden, Wirkungen, ein Verfahren zur Herstellung derselben und diese Verbindungen enthaltende Arzneimittel vorzusehen.

Das Obige wurde überraschenderweise durch die Erfindung erreicht.

Gegenstand der Erfindung sind 2-Methylpropionsäurederivate der allgemeinen Formel

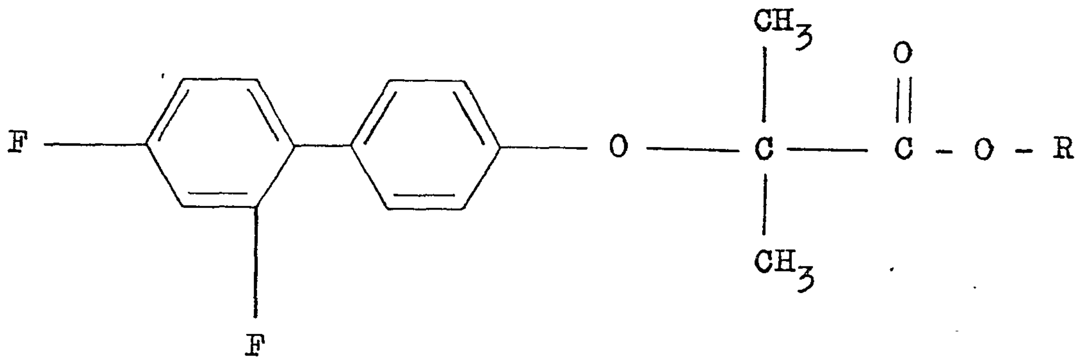

I ,

worin

R für Wasserstoff oder einen Alkylrest mit 1 bis 8 Kohlenstoffatom(en) steht.

Beispiele für Alkylreste, für die R stehen kann, sind Methyl-, Äthyl-, n-Propyl-, Isopropyl-, n-Butyl-, Isobutyl-, sek.-Butyl-, tert.-Butyl-, n-Pentyl-, Isopentyl-, n-Hexyl-, 2-Methyl-n-pentyl-, 3-Methyl-n-pentyl-, 2,2-Dimethyl-n-butyl-, n-Heptyl-, 2,3-Dimethyl-n-pentyl-, 2,4-Dimethyl-n-pentyl-, 2,2,3-Trimethyl-n-butyl-, n-Octyl-, 3,3-Dimethyl-n-hexyl-, 2,2,3,3-Tetramethyl-n-butyl-, 2-Methyl-n-heptyl-, 3-Methyl-n-heptyl- und 2,3,4-Trimethyl-n-pentylreste.

Vorzugsweise ist der Alkylrest, für den R stehen kann, ein solcher mit 1 bis 4, insbesondere 1 oder 2, Kohlenstoffatom(en).

Eine besonders bevorzugte erfindungsgemäße Verbindung ist 2-(2'',4''-Difluordiphenyl-4'-yloxy)-2-methylpropionsäureäthylester.

Die erfindungsgemäßen Verbindungen können nach aus dem chemischen Schrifttum an sich bekannten Verfahren hergestellt werden.

So ist Gegenstand der Erfindung auch ein Verfahren zur Herstellung der erfindungsgemäßen Verbindungen, welches dadurch gekennzeichnet ist, daß in an sich bekannter Weise 1 Moläquivalent 4-(2',4'-Difluorphenyl)-phenol der Formel

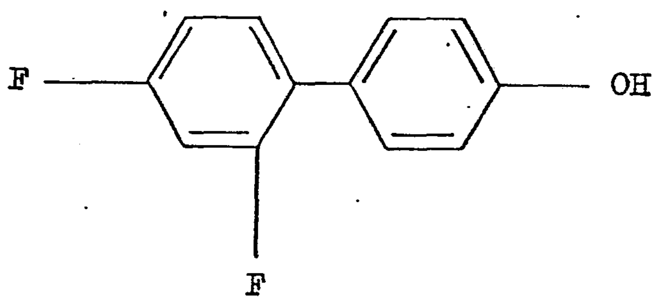

II ,

mit einem Überschuß von Aceton, von einem starken alkalischen Mittel und von Chloroform, jeweils über die Moläquivalentmenge, bei der Siedetemperatur der Reaktionsmischung umgesetzt wird und gegebenenfalls das erhaltene 2-Methylpropionsäurederivat der allgemeinen Formel I, bei welchem R für Wasserstoff steht, in einen Alkylester desselben der allgemeinen Formel I, in welcher R für einen Alkylrest mit 1 bis 8 Kohlenstoffatomen steht, überführt wird.

Das erfindungsgemäße Verfahren kann zweckmäßig wie folgt durchgeführt werden.

Zuerst wird das 4-(2',4'-Difluorphenyl)-phenol der Formel II mit dem Aceton in Gegenwart des starken alkalischen Mittels kondensiert und anschließend wird die Reaktionsmischung zunächst mit dem Chloroform und dann mit Wasser behandelt. Beispiele für starke alkalische Mittel sind Alkali- und Erdalkalimetallhydroxyde. Zwar können die Mengenverhältnisse der verschiedenen Reaktionsteilnehmer innerhalb weiter Grenzen ohne Beeinträchtigung des Ganges oder der Ausbeuten der Reaktion variiert werden, es wurde jedoch festgestellt, daß zweckmäßig je Mol des 4-(2',4'-Difluorphenyl)-phenoles der allgemeinen Formel II etwa 70 bis 90 Moläquivalente Aceton zu verwenden sind, so daß das letztere als Lösungsmittel für die Reaktion wirkt, und etwa 5 bis 8 Moläquivalente des starken alkalischen Mittels einzusetzen sind. Zur Umsetzung wird die Reaktionsmischung aus diesen beiden Reaktionsteilnehmern zum Sieden gebracht und es wird ein schwacher Molüberschuß Chloroform, ebenfalls bezogen auf den Ausgangsstoff 4-(2',4'-Difluorphenyl)-phenol der allgemeinen Formel II, zugesetzt und das Ganze wird etwa 5 bis 7 Stunden zum Sieden erhitzt. Nach dem Abdestillieren eines Teiles des überschüssigen Lösungsmittels (Acetones) unter Vakuum wird Wasser zugesetzt und die Destillation des Lösungsmittels (Acetones) wird bei einer Temperatur von etwa 70 bis 85°C, vorzugsweise 75 bis 80°C, fortgesetzt. Das Endprodukt wird dann aus der Reaktionsmischung mittels bekannter Verfahrensweisen gewonnen.

Die Überführung des so erhaltenen 3-Methylpropionsäurederivates der allgemeinen Formel I, bei welchem R für Wasserstoff steht, das heißt von 2-(2'',4''-Difluordiphenyl-4'-yloxy)-2-methylpropionsäure, in ihre Alkylester der allgemeinen Formel I, in welcher R für einen Alkylrest mit

1 bis 8 Kohlenstoffatomen steht, kann nach an sich bekannten Veresterungsverfahrensweisen durchgeführt werden. Beispielsweise kann eine unmittelbare Veresterung der ersteren mit Alkanolen mit 1 bis 8 Kohlenstoffatomen in Gegenwart von starken Säuren, wie Schwefelsäure, Salzsäure, p-Toluolsulfonsäure oder Bortrifluorid, oder eine Umwandlung der ersteren in das entsprechende Acylhalogenid und anschließende Umsetzung mit einem Alkanol mit 1 bis 8 Kohlenstoffatomen vorgenommen werden. Auch in diesem Fall können die erhaltenen Endprodukte aus der Reaktionsmischung nach bekannten Verfahrensweisen gewonnen werden.

Das im erfindungsgemäßen Verfahren als Ausgangsstoff verwendete 4-(2'',4''-Difluorphenyl)-phenol der Formel II kann nach dem in der deutschen Offenlegungsschrift 2 532 559 beschriebenen Verfahren erhalten worden sein.

Ferner sind erfindungsgemäß Arzneimittel, welche 1 oder mehr der erfindungsgemäßen Verbindungen als Wirkstoff beziehungsweise Wirkstoffe, gegebenenfalls zusammen mit 1 oder mehr üblichen festen oder flüssigen pharmazeutischen Trägern und/oder Hilfsstoffen, enthalten, vorgesehen. Die erfindungsgemäßen Verbindungen haben nämlich wie bereits erwähnt beträchtliche pharmakologische, insbesondere den Lipidgehalt und Cholesteringehalt im Blut senkende, Wirkungen.

Diese Wirkungen wurden gegenüber der in der Therapie der Hyperlipidämie (übermäßiger Lipidgehalt im Blut) und Hypercholesterinämie (übermäßiger Cholesteringehalt im Blut) verbreitet angewandten und anerkannt gut wirksamen bekannten Vergleichssubstanz 2-(4'-Chlorphenoxy)-2-methylpropionsäure [Clofibrat] bestimmt.

Es wurden vorherbestimmte tägliche Dosen einer erfindungsgemäßen Verbindung Laboratoriumstieren (üblicherweise in Gruppen von je 6 Tiere unterteilten Ratten) peroral verabreicht, während die Vergleichssubstanz 2-(4'-Chlorphenoxy)-2-methylpropionsäure [Clofibrat] in einer Dosis von 130 mg/kg/Tag ebenfalls peroral verabreicht wurde. Die Behandlung wurde 10 Tage fortgesetzt, dann wurden die Tiere geopfert und ihr Blut wurde gesammelt und der Bestimmung seines Triglycerid- und Cholesteringehaltes unterzogen. Die erhaltenen Ergebnisse, ausgedrückt als prozentuale Verminderung des Triglycerid- und Cholesteringehaltes im Blut (prozentuale Hemmung der Bildung des Triglycerid- und Cholesteringehaltes im Blut) gegenüber den Blindversuchen (das heißt den Ratten, welchen weder eine erfindungsgemäße Verbindung noch die Vergleichssubstanz verabreicht wurde), sind in der folgenden Tabelle zusammengestellt.

Tabelle

| Verbindung Beispiel | Verbindung Bezeichnung | Perorale Dosis in mg/kg | Zahl der Ratten | Verminderung des Triglyceridgehaltes in % | Verminderung des Cholesteringehaltes in % |
|---|---|---|---|---|---|
| 2 | 2 | 2,5 | 6 | 22 | 11 |
| 2 | 2 | 35 | 6 | 36 | 23 |
| 2 | 2 | 50 | 6 | 29 | 30 |
| Vergleichssubstanz | 2-(4'-Chlorphenoxy)-2-methylpropionsäure [Clofibrate] | 130 | 6 | 21 | 24 |

Die in der obigen Tabelle zusammengestellten Ergebnisse zeigen eindeutig, daß die erfindungsgemäße Verbindung ihre Wirksamkeit bezüglich der Hemmung der Biosynthese der Triglyceride und von Cholesterin bei bei weitem niedrigeren Dosen als die Vergleichssubstanz 2-(4'-Chlorphenoxy)-2-methylpropionsäure [Clofibrat] entfaltet.

Die beträchtlichen den Lipidgehalt und den Cholesteringehalt im Blut senkenden Wirkungen der erfindungsgemäßen Verbindungen wurden in weiteren Versuchen bestätigt. In diesen wurden erfindungsgemäße Verbindungen 20 beziehungsweise 30 Tage in vorherbestimmten Molmengen verabreicht, wobei wiederum 2-(4'-Chlorphenoxy)-2-methylpropionsäure [Clofibrat] als Vergleichssubstanz in denselben Molmengen wie die erfindungsgemäßen Verbindungen verabreicht wurde. Als repräsentatives Beispiel wurde festgestellt, daß bei der Dosis von 0,5 Millimol/kg die erfindungsgemäßen Verbindungen hinsichtlich der Senkung des Triglycerid- und Cholesteringehaltes im Blut etwa 3-mal so wirksam wie die Vergleichssubstanz 2-(4'-Chlorphenoxy)-2-methylpropionsäure [Clofibrat] waren.

Diese beachtlichen pharmakologischen Eigenschaften sind mit einer ziemlich niedrigen Toxizität gepaart, indem die peroralen $LD_{50}$-Werte der erfindungsgemäßen Verbindungen an Mäusen gut über 1 000 mg/kg liegen. In Anbetracht der niedrigen Dosen, bei welchen die erfindungsgemäßen Verbindungen ihre obigen Wirkungen zeigen, ergibt sich, daß sie äußerst günstige therapeutische Indices haben, was sie als Arzneimittel von hoher Sicherheit ausweist.

Auf Grund des Obigen können die erfindungsgemäßen Verbindungen vorteilhaft zur Behandlung von Hyperlipidämie

und Hypercholesterinämie angewandt werden, wobei sie den Lipidgehalt und Cholesteringehalt im Blut auf normale Werte bringen.

Die erfindungsgemäßen Verbindungen können in Form von Arzneimittelpräparaten vorliegen und therapeutisch angewandt werden.

Die erfindungsgemäßen Verbindungen beziehungsweise Arzneimittelpräparate können auf verschiedenen Wegen, beispielsweise peroral, intravenös oder intramuskulär, verabreicht werden, wobei die perorale Verabreichung bevorzugt ist. So können die erfindungsgemäßen Arzneimittelpräparate zur peroralen Verabreichung in Form von Tabletten, dispergierbaren Pulvern, Kapseln, Pillen, mit Zucker überzogenen Tabletten, Körnern beziehungsweise Granulaten, Sirupen, Elixieren oder Lösungen vorliegen.

Die Arzneimittelpräparate zur peroralen Verabreichung können 1 oder mehr übliche Hilfsstoffe, beispielsweise Süßungsmittel, Schmackhaftmachungsmittel beziehungsweise Aromastoffe, Farbstoffe, Überzugsmittel und Konservierungsmittel, enthalten, um so ein gefälliges und schmackhaftes Arzneimittelpräparat zu haben. Die Tabletten können den erfindungsgemäßen Wirkstoff beziehungsweise die erfindungsgemäßen Wirkstoffe in Mischung mit herkömmlichen pharmazeutischen Trägern beziehungsweise Excipienten, beispielsweise inerten Verdünnungsmitteln, wie Calciumcarbonat, Natriumcarbonat, Milchzucker und/oder Talk, Granuliermitteln und/oder das Zerfallen fördernden Mitteln, wie Stärke, Alginsäure und/oder Natriumcarboxymethylcellulose, Bindemitteln, wie Stärke, Gelatine, Gummi arabicum und/oder

Polyvinylpyrrolidon, und/oder Gleitmitteln, wie Magnesiumstearat, Stearinsäure und/oder Talk, enthalten.

Auch die Sirupe, Elixiere und Lösungen können in an sich bekannter Weise hergestellt sein. Sie können zusammen mit dem erfindungsgemäßen Wirkstoff beziehungsweise den erfindungsgemäßen Wirkstoffen Suspendiermittel, wie Methylcellulose, Hydroxyäthylcellulose, Traganthgummi und/oder Natriumalginat, und/oder Netzmittel, wie Lecithin, Polyoxyäthylenstearate und/oder Polyoxyäthylensorbitanmonooleat, und/oder übliche Konservierungsmittel, Süßungsmittel und/oder Puffermittel enthalten.

Die Kapseln beziehungsweise mit Zucker überzogenen Tabletten können den erfindungsgemäßen Wirkstoff beziehungsweise die erfindungsgemäßen Wirkstoffe allein oder in Mischung mit festen inerten Verdünnungsmitteln, wie Calciumcarbonat, Calciumphosphat und/oder Kaolin, enthalten.

Die Wirkstoffdosis zur Bekämpfung der Hyperlipidämie und Hypercholesterinämie kann innerhalb weiter Grenzen je nach dem erfindungsgemäßen Wirkstoff variieren. Im allgemeinen werden gute Ergebnisse mit täglichen Dosen der erfindungsgemäßen Verbindungen von etwa 1 bis 10 mg/kg Körpergewicht erzielt.

Die erfindungsgemäßen Arzneimittelpräparate enthalten den erfindungsgemäßen Wirkstoff beziehungsweise die erfindungsgemäßen Wirkstoffe zweckmäßig in Mengen von etwa 10 bis 100 mg. Sie eignen sich für tägliche Einzelverabreichungen oder mehrfache Verabreichungen.

Die erfindung wird an Hand der folgenden Beispiele näher erläutert.

## Beispiel 1

2-(2'',4''-Difluordiphenyl-4'-yloxy)-2-methylpropionsäure

Es wurde eine Mischung von 41 g (0,2 Mol) 4-(2',4'-Difluorphenyl)-phenol, 1 200 $cm^3$ (16,3 Mol) Aceton und 45 g (1,125 Mol) Natriumhydroxyd in Flocken zum Sieden gebracht und ihr wurden 31 g (0,26 Mol) Chloroform zugetropft. Die Zugabe nahm etwa 15 Minuten in Anspruch. Die Mischung wurde etwa 6 Stunden lang unter Rückfluß zum Sieden erhitzt, worauf das Aceton unter Vakuum abgedampft wurde. Etwa am Ende der Destillation wurden 500 $cm^3$ Wasser zugesetzt und die Destillation wurde unter Atmosphärendruck fortgesetzt.

Nach dem Kühlen auf Raumtemperatur wurde der pH-Wert der Reaktionsmischung mit Hilfe einer 50%-igen wäßrigen Salzsäure auf 2 gebracht und der erhaltene Niederschlag wurde durch Filtrieren gewonnen und anschließend mit 1 000 $cm^3$ Chloroform und 500 $cm^3$ einer 10%-igen wäßrigen Natriumbicarbonatlösung versetzt. Die erhaltene Mischung wurde etwa 30 Minuten unter Rückfluß zum Sieden erhitzt und dann auf 25°C gekühlt, wodurch sich 2 Phasen trennten, nämlich eine organische und eine anorganische. Die beiden Phasen wurden gewonnen, die organische Phase wurde mit 100 $cm^3$ einer 10%-igen wäßrigen Natriumbicarbonatlösung gewaschen und die beiden wäßrigen Phasen wurden miteinander vereinigt und nach dem Waschen mit 100 $cm^3$ Chloroform, Entfärben mit 5 g Holzkohle und Filtrieren langsam mit einer 50%-igen wäßrigen Salzsäure auf einen pH-Wert von 2 angesäuert. Der erhaltene Niederschlag wurde durch Filtrieren gewonnen und aus Äthylalkohol umkristallisiert.

So wurden als Produkt 45 g (77,1% der Theorie, bezogen auf das eingesetzte 4-(2',4'-Difluorphenyl)-phenol 2-(2'',4''-Difluordiphenyl-4'-yloxy)-2-methylpropionsäure mit einem Schmelzpunkt von 174 bis 175°C erhalten.

Beispiel 2

2-(2'',4''-Difluordiphenyl-4'-yloxy)-2--methylpropionsäureäthylester

Es wurden einer Lösung von 29 g (0,1 Mol) der wie im Beispiel 1 beschrieben erhaltenen 2-(2'',4''-Difluordi-diphenyl-4'-yloxy)-2-methylpropionsäure in 200 $cm^3$ wasserfreiem Äthylalkohol 2,2 $cm^3$ konzentrierte Schwefelsäure zugetropft und die erhaltene Mischung wurde 5 Stunden lang unter Rückfluß zum Sieden erhitzt. Nach dem Abdampfen des Alkoholes unter Vakuum bei einer Temperatur von nicht mehr als 50°C wurde der Rückstand in 150 $cm^3$ Methylenchlorid und 150 $cm^3$ Wasser aufgenommen. Die beiden Phasen wurden getrennt, die organische Phase wurde mit einer 5%-igen wäßrigen Natriumhydroxydlösung gewaschen, über Natriumsulfat getrocknet und zur Trockne eingedampft. Es wurde ein öliger Rückstand erhalten, welcher unter einem Vakuum von 2 mm Hg destilliert wurde.

So wurden 25 g (78,1% der Theorie bezogen auf die eingesetzte 2-(2'',4''-Difluordiphenyl-4'-yloxy)-2-methylpropionsäure) 2-(2'',4''-Difluordiphenyl-4'-yloxy)--2-methylpropionsäureäthylester mit einem Siedepunkt von 164 bis 166°C/2 mm Hg erhalten.

Nach der obigen Verfahrensweise konnten mit den folgenden Alkoholen die entsprechenden Ester der 2-(2'',4''-Difluordiphenyl-4'-yloxy)-2-methylpropionsäure erhalten werden: Methanol, n-Propanol, Isopropanol, n-Butanol, Isobutanol, tert.-Butanol, n-Pentanol, Isopentanol, n-Hexanol, 2-Methyl-n-pentan-1-ol, 3-Methylpentan-1-ol, n-Heptanol, 2,3-Dimethyl-n-pentan-1-ol, 2,2,3-Trimethyl-n-butanol, n-Octanol, 3,3-Dimethyl-n-hexanol, 2-Methyl-n-heptan-1-ol und 2,3,4-Trimethyl-n-pentan-1-ol.

## Beispiel 3

Es wurde eine mit Zucker überzogene Tablette in an sich bekannter Weise aus den folgenden Bestandteilen hergestellt:

| | | |
|---|---|---|
| 60 mg | 2-(2'',4''-Difluordiphenyl-4'-yloxy)-2-methylpropionsäureäthylester | |
| 15 mg | Natriumcarboxymethylcellulose | |
| 15 mg | Magnesiumstearat | |
| 10 mg | Gelatine | und |
| 35 mg | Rohrzucker | |

sowie

Gummi arabicum, Milchzucker, Titandioxyd und Aluminiumlack.

Beispiel 4

Es wurde eine Kapsel in an sich bekannter Weise aus den folgenden Bestandteilen hergestellt:

| | | |
|---|---|---|
| | 50 mg | 2-(2'',4''-Difluordiphenyl-4'-yloxy)--2-methylpropionsäureäthylester |
| | 10 mg | Talk |
| | 10 mg | Milchzucker |
| zum Auffüllen auf | 150 mg | Stärke |

Patentansprüche

Patentansprüche

1.) 2-Methylpropionsäurederivate der allgemeinen Formel

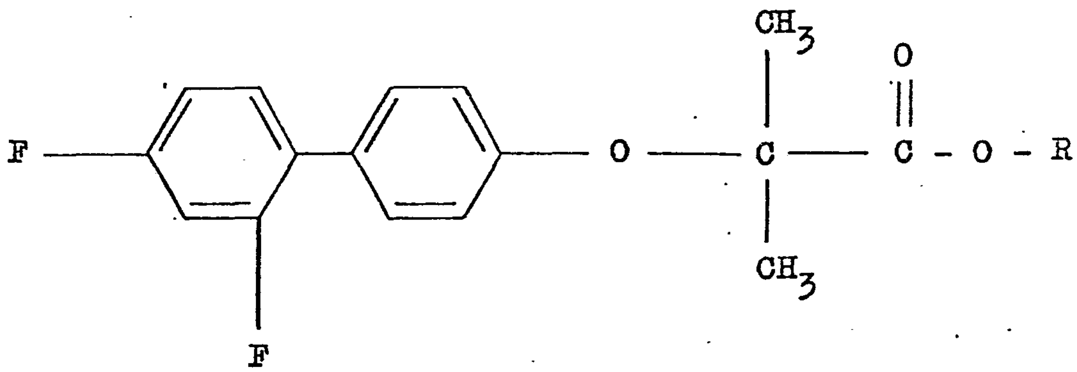

I,

worin

R für Wasserstoff oder einen Alkylrest mit 1 bis 8 Kohlenstoffatom(en) steht.

2.) 2-Methylpropionsäurederivate nach Anspruch 1, dadurch gekennzeichnet, daß der Alkylrest, für den R stehen kann, ein solcher mit 1 bis 4, insbesondere 1 oder 2, Kohlenstoffatom(en) ist.

3.) 2-(2'',4''-Difluordiphenyl-4'-yloxy)-2-methylpropionsäureäthylester.

4.) Verfahren zur Herstellung der Verbindungen nach Anspruch 1 bis 3, dadurch gekennzeichnet, daß man in an sich bekannter Weise 1 Moläquivalent 4-(2',4'-Difluorphenyl)-phenol der Formel

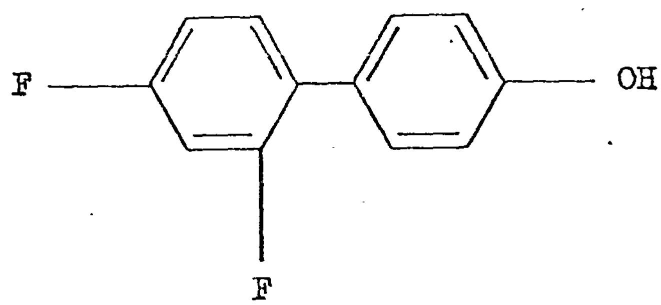

II

mit einem Überschuß von Aceton, von einem starken alkalischen Mittel und von Chloroform, jeweils über die Moläquivalentmenge, bei der Siedetemperatur der Reaktionsmischung umsetzt und gegebenenfalls das erhaltene 2-Methylpropionsäurederivat der allgemeinen Formel I, bei welchem R für Wasserstoff steht, in einen Alkylester desselben der allgemeinen Formel I, in welcher R für einen Alkylrest mit 1 bis 8 Kohlenstoffatomen steht, überführt.

5.) Arzneimittel, gekennzeichnet durch einen Gehalt an 1 oder mehr Verbindungen nach Anspruch 1 bis 3 als Wirkstoff beziehungsweise Wirkstoffen, gegebenenfalls zusammen mit 1 oder mehr üblichen festen oder flüssigen pharmazeutischen Trägern und/oder Hilfsstoffen.

Zusammenfassung

Europäisches Patentamt

**EUROPÄISCHER RECHERCHENBERICHT**

0033980

Nummer der Anmeldung
EP 81100990.1

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | US - A - 3 652 646 (T. LEIGH et al.)<br>+ Spalten 1-3,6 +<br>& DE-C- 1593 907<br>-- | 1,5 | C 07 C 59/68<br>C 07 C 69/712<br>A 61 K 31/19<br>A 61 K 31/215<br>C 07 C 51/00<br>C 07 C 67/00 |
| | CHEMICAL ABSTRACTS, Band 50, 1956, Columbus, Ohio, USA<br>M. JULIA, M. BAILLARGE, G. TCHERNOFF "Aryloxyisobutyric acid derivatives"<br>Spalte 15458, Zusammenfassung e,f<br>& Bull.soc.chim. France 1956, 776-83<br>---- | 1,4 | |

RECHERCHIERTE SACHGEBIETE (Int. Cl.³)

C 07 C 59/00
C 07 C 69/00
A 61 K 31/00
C 07 C 51/00
C 07 C 67/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 15-04-1981 | HOFBAUER |